# EUROPÄISCHE PATENTSCHRIFT

(11) **EP 1 097 209 B1**
(45) Veröffentlichungstag und Bekanntmachung des Hinweises auf die Patenterteilung: **27.10.2004**
(21) Anmeldenummer: 99960680.9
(22) Anmeldetag: 13.07.1999
(51) Int. Cl.: C12N 15/12, A61K 38/17, A61K 48/00, G01N 33/50

(54) **HEMMUNG VON ALOPEZIE**
INHIBITION OF ALOPECIA
INHIBITION DE L'ALOPECIE

(30) Priorität: 13.07.1998 DE 19831043
(43) Veröffentlichungstag der Anmeldung: 09.05.2001
(73) Patentinhaber: Deutsches Krebsforschungszentrum Stiftung des Öffentlichen Rechts, 69129 Heidelberg (DE)
(72) Erfinder: BOEHM, Thomas, D-79279 Vorstetten (DE); SCHLAKE, Thomas, D-79194 Gundelfingen (DE); MEIER, Natalia, D-79104 Freiburg (DE)
(74) Vertreter: Schüssler, Andrea, Dr.
(86) Internationale Anmeldenummer: PCT/DE1999/002185
(87) Internationale Veröffentlichungsnummer: WO 2000/002908

(56) Entgegenhaltungen:
- DD-A- 58 358
- DE-C- 19 736 198
- SCHÜDDEKOPF ET AL: "THE WHN TRANSCRIPTION FACTOR ENCODED BY THE NUDE LOCUS CONTAINS AN EVOLUTIONARY CONSERVED AND FUNCTIONALLY INDISPENSABLE ACTIVATION DOMAIN" PROCEEDINGS OF THE NATIONAL ACADEMY OF SCIENCES, USA, Bd. 93, 1996, Seiten 9661-9664, XP002130551
- KUROOKA H ET AL: "Rescue of the hairless phenotype in nude mice by transgenic insertion of the wild-type Hfh11 genomic locus." INTERNATIONAL IMMUNOLOGY, (1996 JUN) 8 (6) 961-6. , XP000876588
- KAIN S R ET AL: "GREEN FLUORESCENT PROTEIN AS A REPORTER OF GENE EXPRESSION AND PROTEIN LOCALIZATION" BIOTECHNIQUES,US,EATON PUBLISHING, NATICK, Bd. 19, Nr. 4, 1. Oktober 1995 (1995-10-01), Seiten 650-655, XP002033687 ISSN: 0736-6205
- FINK, P. ET AL: "A cDNA encoding the human type I hair keratin hHa1" BIOCHIM. BIOPHYS. ACTA (1995), 1264(1), 12-14 , XP000876612
- SHORPP ET AL: "CHARACTERIZATION OF MOUSE AND HUMAN NUDE GENES" IMMUNOGENETICS, Bd. 46, 1997, Seiten 509-515, XP000876601 in der Anmeldung erwähnt

## Beschreibung

Die vorliegende Erfindung betrifft ein Verfahren zur Hemmung von Alopezie und ein System zur Identifizierung von Alopezie hemmenden Substanzen.

Alopezie ist eine weit verbreitete Erkrankung des Haares, bei der vollständiger Haarverlust eintreten kann. Die Ursachen von Alopezie sind nicht bekannt. Insofern ist es auch nicht möglich, gezielt in diese Erkrankung einzugreifen.

DD-A-58358 betrifft ein Verfahren zur Herstellung eines Keratin-Partial-Hydrolysats mit geringem Hydrolysegrad, wobei Human-Haare mittels geringer Mengen schwach konzentrierter anorganischer Säuren hydrolysiert werden.

Der vorliegenden Erfindung liegt somit die Aufgabe zugrunde ein Mittel bereitzustellen, mit dem dieses erreicht werden kann.

Erfindungsgemäß wird dies durch die Gegenstände der Patentansprüche erreicht.

Die vorliegende Erfindung beruht auf den Erkenntnissen des Anmelders, daß bestimmte Formen der Alopezie auf einer gestörten Keratinisierung des Haares beruhen. Ferner hat er erkannt, daß bei Alopezie die mRNA verschiedener Gene, nicht vorhanden, z.B. des Ha3-Gens, oder unterrepräsentiert, z.B. der Ha1-, Ha2- und Ha4-Gene, ist (vgl. Figuren 1 und 2). Die Genprodukte der Ha1-, Ha2-, Ha3- und Ha4-Gene sind Haarkeratine. Der Anmelder hat gefunden, daß die Expression des Ha3-Gens durch ein Genprodukt des whn-Gens reguliert wird. Insbesondere hat er gefunden, daß durch Expression des whn-Gens die Expression des Ha3-Gens induziert werden kann (vgl. Fig. 3). Auch hat er gefunden, daß die Expression anderer Haarkeratin-Gene durch das Genprodukt des whn-Gens wesentlich beeinflußt wird. Der Anmelder hat weiterhin gefunden, daß die Expression des whn-Gens im Verlauf des Haarzyklus schwankt.

Insbesondere hat er gefunden, daß die whn-Expression in der Telogenphase des Haarzyklus auf nicht mehr detektierbare Spiegel absinkt. Ferner hat er gefunden, daß das whn-Gen von zwei Promotoren transkribiert werden kann. Der Anmelder hat seine Erkenntnisse mit Hilfe von Nacktmäusen und Hela-Zellen gewonnen.

Erfindungsgemäß werden die Erkenntnisse des Anmelders für ein Arzueimiltel zur Hemmung von Alopezie genutzt, das die Erhöhung der zellulären Menge von Haarkeratinen umfaßt.

Der Ausdruck "Erhöhung der zellulären Menge von Haarkeratinen" weist darauf hin, daß in Zellen die Menge von ein oder mehreren Haarkeratinen, insbesondere von Ha1, Ha2, Ha3 und Ha4, die gering oder gar nicht vorhanden sein kann, erhöht wird. Dies kann durch übliche Verfahren bzw. Substanzen erreicht werden. Beispielsweise können den Zellen ein oder mehrere Haarkeratine, insbesondere Ha1, Ha2, Ha3 und Ha4, als solche oder in Form von sie kodierender DNA zugegeben werden. Die DNA kann in üblichen Expressionsvektoren vorliegen. Auch können Substanzen zugegeben werden, welche die Expression von ein oder mehreren Haarkeratinen, insbesondere von Ha1, Ha2, Ha3 und Ha4, aktivieren. Solche Substanzen sind z.B. das Genprodukt des whn-Gens oder eine hierfür kodierende DNA. Diese kann in üblichen Expressionsvektoren vorliegen. Ferner können Substanzen zugegeben werden, welche die Expression des whn-Gens aktivieren. Diese können ebenfalls als solche oder in Form von sie kodierender DNA vorliegen, wobei letztere auch in üblichen Expressionsvektoren vorliegen kann. Der Ausdruck "Zellen" umfaßt Zellen jeglicher Art und Abstammung. Ferner umfaßt er Gewebe und Organismen, insbesondere Tiere und den Menschen.

Die Verabreichung von Substanzen, die Alopezie hemmen, kann in üblicher Weise, vorzugsweise lokal erfolgen. Auch können die Substanzen in üblichen Formulierungen vorliegen. Bei lokaler Verabreichung der Substanzen eignen sich z.B. Cremes, Salben, Shampoos und Haarwasser. Auch können die Substanzen in Partikeln vorliegen, die leicht aufgenommen werden. Beispiele solcher Partikel sind Liposome. Der Fachmann kennt Verfahren, um für die einzelnen Substanzen die geeigneten Formulierungen bzw. Verabreichungsformen zu finden.

Ein weiterer Gegenstand der vorliegenden Erfindung ist ein Verfahren zur Identifizierung von Substanzen, die sich zur Hemmung von Alopezie eignen. Ein solches Verfahren umfaßt die Bestimmung der Erhöhung der zellulären Menge von Haarkeratinen und/oder der Erhöhung ihrer Genexpression und/oder der Erhöhung des Genprodukts des whn-Gens. Insbesondere umfaßt das System nicht menschliche Tiere oder Zellen, wobei Zellen bevorzugt sind, in denen ein oder mehrere exprimierbare Haarkeratin-Gene und/oder ein oder mehrere exprimierbare Gene, deren Genprodukte die Genexpression von Haarkeratinen aktivieren, jeweils fusioniert mit einem Reporter-Gen vorliegen. Die verwendeten Zellen, in denen besagte Gene fusioniert mit einen Reporter-Gen vorliegen, umfassen nicht den Menschen. Die Haarkeratin-Gene können insbesondere jene von Ha1, Ha2, Ha3 und Ha4 sein. Die die Genexpression von Haarkeratinen aktivierende Substanz stellt ein Genprodukt des whn-Gens dar. Desweiteren können die vorstehenden Gene eine Wildtyp- oder eine veränderte Sequenz aufweisen, wobei sich letztere von der Wildtyp-Sequenz durch ein oder mehrere Basenpaare unterscheiden kann. Die Unterschiede können in Form von Additionen, Deletionen, Substitutionen und/oder Inversionen von Basenpaaren vorliegen. Ferner kann ein vorstehendes Reporter-Gen jegliches sein, insbesondere kann es für ein Enzym, z.B. alkalische Phosphatase, oder ein fluoreszierendes Protein, z.B. GFP, kodieren. Desweiteren können die Fusionsgene extrachromosomal vorliegen oder im Zell-Genom, insbesondere anstelle eines oder beider Allele der Haarkeratine und/oder der Gene, deren Genprodukte die Expression von Haarkeratinen aktivieren. Ferner kann das System Stoffe enthalten, die sich zum Nachweis der exprimierten Haarkeratine und/oder von ihre Genexpression aktivierenden Substanzen bzw. der Fusionsgene, eignen. Solche Stoffe können sich zum Nachweis auf dem Nukleinsäure- bzw. Protein-Level eignen.

Mit der vorliegenden Erfindung ist es möglich Alopezie zu hemmen. Ferner ist es möglich Alopezie zu diagnostizieren, in dem z.B. die Genexpression von Haarkeratinen und/oder von Substanzen bestimmt wird, welche diese aktivieren. Des weiteren ist es möglich Substanzen zu finden, die sich zur Hemmung von Alopezie eignen. Hierfür wird ein System bereitgestellt, das sich zum schnellen und zuverlässigen Screenen von verschiedensten Substanzen eignet. Damit stellt die vorliegende Erfindung Mittel bereit eine weit verbreitete Erkrankung des Haares zu diagnostizieren und zu therapieren.

### Kurze Beschreibung der Zeichnungen:

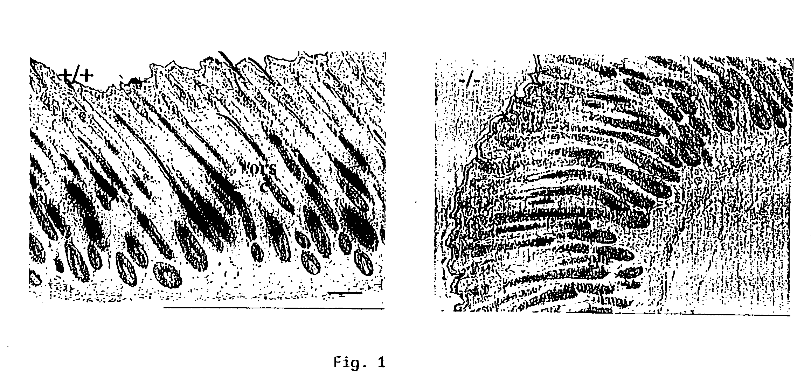

- Fig. 1: zeigt eine in situ RNA-Hybridisierung mit einer Sonde für mHa3 in normalen (whn +/+) und mutanten (whn -/-) Mäusen. Die Transkripte für mHa3 (sichtbar als braune Silberkörner) sind in Haarfollikeln der Nacktmaus nicht nachweisbar. Die Linie entspricht 100 µm.

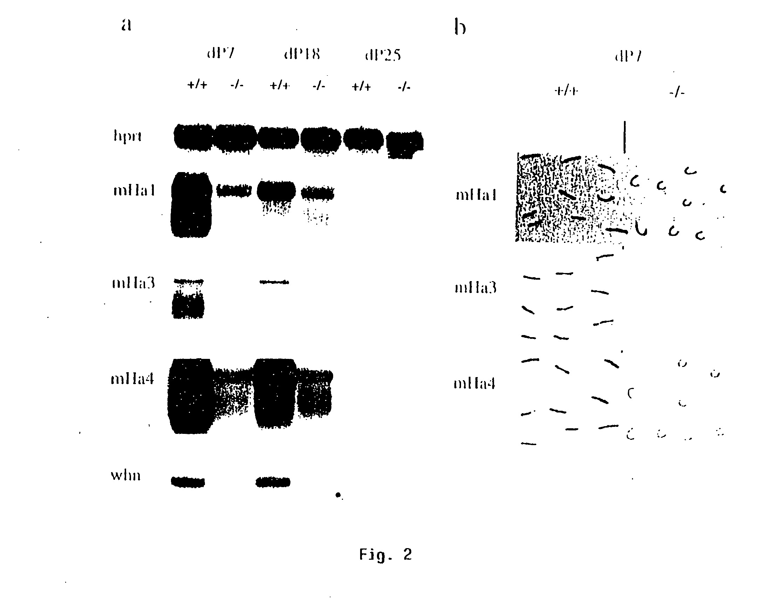

- Fig. 2: zeigt die Expression von whn und Haarkeratinen im Haarfollikel der Maus.
A. Northern Filter-Hybridisierung mit RNA aus Gesamthaut von normalen Mäusen (whn +/+) und Nacktmäusen (whn -/-) mittels Sonden für hprt- und whn-Gene sowie Ha1-, Ha3-, Ha4-Gene zu drei Zeitpunkten nach der Geburt dP7, 7 Tage nach Geburt etc.).
B. In situ RNA-Hybridisierung in Haut aus normalen (whn +/+) und Nacktmäusen (whn -/-) mit Sonden für Ha1-, Ha3- und Ha4-Gene. Ein Autoradiogramm von Hautschnitten am Tag 7 nach der Geburt ist gezeigt.

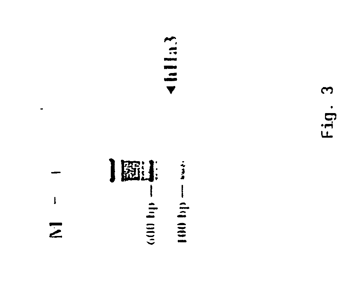

- Fig. 3: zeigt die Regulation der Keratin-Gen-Expression. Hela-Zellen wurden mit einem whn-Expressions-Konstrukt transient transfiziert (+) und die Anwesenheit von Ha3-spezifischer mRNA wurde über eine RT-PCR nachgewiesen.Die Molekulargewichtsmarker sind in bp angegeben.

Die vorliegende Erfindung wird durch die nachstehenden Beispiele erläutert.

### Beispiel 1: Nachweis des Verlustes der Expression des Ha3-Gens in Mäusen mit Alopezie.

Es wurde das "Representational Difference Analysis" (RDA)-Verfahren durchgeführt. Dieses Verfahren umfaßt die Isolierung von mRNA aus Hautzellen von (whn +/+)-Mäusen bzw. (whn -/-)-Mäusen (Alopezie aufweisende Mäuse, die keine Expression des whn-Gens aufweisen), die Umschreibung der mRNA in cDNA und die Differenzierung der cDNA, wodurch solche identifiziert wird, die in (whn -/-)-Mäusen unter- bzw. überexprimiert wird.

### A) Sequenz der Oligonukleotidadaptoren

Folgende Oligonukleotidadaptorenpaare wurden für die RDA benötigt:
R-Bgl-12: 5'-GATCTGCGGTGA-3'
R-Bgl-24: 5'-AGCACTCTCCAGCCTCTCACCGCA-3'

R-Bgl-12: 5'-GATCTGTTCATG-3'
R-Bgl-24: 5'-ACCGACGTCGACTATCCATGAACA-3'

N-Bgl-12: 5'-GATCTTCCCTCG-3'
N-Bgl-24: 5'-AGGCAACTGTGCTATCCGAGGGAA-3'

### B) Herstellung von poly A-mRNA aus den miteinander zu vergleichenden Geweben

Zunächst wurde RNA aus der Haut von (whn +/+)-bzw. (whn -/-)-Mäusen nach der "Single-Step RNA-Extraction"-Methode (Chomczynski and Sacchi, 1987) gewonnen. Die poly A-mRNA-Fraktionen aus den beiden RNA-Populationen wurden anschließend mit Hilfe von Dynabeads Oligo(dT) nach dem entsprechenden Protokoll der Firma Dynal isoliert.

### C) Synthese doppelsträngiger cDNA

Zur Synthese von doppelsträngiger (whn +/+)-bzw. (whn -/-)-cDNA wurde das "Ribo Clone cDNA Synthesis Kit" der Firma Promega verwendet. Jeweils 4 µg poly A-mRNA wurden eingesetzt, um ungefähr 2 µg cDNA zu erhalten.

### D) Differenzanalyse

1. Restriktionsverdau der doppelsträngigen cDNAs
   a) Ungefähr 2 µg jeder cDNA wurden in einem 100 µl-Reaktionsansatz mit der Restriktionsendonuklease DpnII 2 h bei 37°C verdaut.
   b) Die Reaktionslösungen wurden anschließend zweimal mit einem Phenol/Chloroform-Gemisch (1:1) und einmal mit 100%igem Chloroform extrahiert.
   c) Die in den wäßrigen Phasen der beiden Reaktionsansätze enthaltene DNA wurde jeweils mit 2 µg Glykogen, 50 µl 10 M Ammoniumacetat und 650 µl 100% Ethanol versetzt und 20 min auf Eis gefällt.

   Nach 14-minütiger Zentrifugation bei 4°C und 14000 upm wurde der Überstand verworfen und das DNA-Pellet mit 70% Ethanol gewaschen. Nach erneuter Zentrifugation und Entfernung der alkoholischen Phase wurde die getrocknete DNA in 20 µl TE-Puffer resuspendiert.
2. Ligation der cDNAs an das R-Bgl-Oligonukleotidadaptorenpaar
   a) In einem Reaktionsgefäß wurden vereinigt: 20 µl geschnittene cDNA (gesamter Reaktionsansatz aus Punkt D)lc)
      8 µg R-Bgl-24
      4 µg R-Bgl-12
      6 µl 10 x Ligase Puffer
      x µl Wasser
      57 µl Endvolumen
   b) Der Reaktionsansatz wurde in einem Thermocycler (Peltier Thermocycler PTC-200, MJ Research) auf 50°C erhitzt, 1 min auf dieser Temperatur gehalten und dann im Laufe einer Stunde wieder auf 10°C abgekühlt (ramp rate: 0,1°C/9 sec).
   c) Nach Hinzufügen von 3 µl T4 DNA Ligase (1 U/µl) wurde das Gemisch über Nacht bei 16°C inkubiert.
3. Synthese von "Repräsentationen" der miteinander zu vergleichenden cDNA-Populationen
   a) Zur Generierung sog. "Repräsentationen" der ligierten cDNAs wurde zunächst das Volumen der Ligationsansätze aus Punkt 2c) durch Zugabe von jeweils 140 µl Wasser auf 200 µl ergänzt.
      Aus dieser verdünnten Lösung wurden dann pro cDNA-Population (whn +/+)- bzw. (whn - /-)-Haut 30 Reaktionen zu jeweils 200 µl angesetzt.
      Einem solchen Ansatz wurden der Reihe nach folgende Reaktanden zugegeben:
      143 µl Wasser
      20 µl 10x PCR-Puffer
      20 µl 2 mM dNTPs
      10 µl 25 mM Mg-Chlorid
      2 µl R-Bgl-24 (1 µg/µl)
      4 µl verdünnter Ligationsansatz
   b) PCR:
      3 min: 72°C
      Hinzufügen von 1 µl Taq-DNA-Polymerase (5 U/µl)
      20 x: 5 min: 95°C 3 min: 72°C
      zuletzt: Abkühlen auf 4°C.
   c) Zur Aufbereitung der Reaktionslösungen wurden jeweils 4 Reaktionsansätze in einem Gefäß vereinigt.
      Extraktion: 2 x mit jeweils 700 µl Phenol/Chloroform (1:1), 1 x mit Chloroform 100%;
      Fällung: Zugabe von 75 µl 3 M Na-Acetatlösung (pH 5,3) und 800 µl 2-Propanol zu jedem Reaktionsgefäß, 20 min Eis.
      Zentrifugation: 14 min, 14000 rpm, 4°C.
      Waschen des DNA-Pellets mit Ethanol 70% und Resuspension in soviel Wasser, daß eine Konzentration von 0, 5 µg/µl resultierte.
4. Restriktionsverdau der "Repräsentationen"
   a) Zur Entfernung der R-Bgl-Oligonukleotidadaptoren wurden 300 µg jeder Repräsentation (whn +/+)-Haut bzw. (whn -/-)-Haut einem Restriktionsverdau unterzogen. Es wurde nach Zugabe der folgenden Reaktanden 4 h bei 37°C inkubiert:
      600 µl cDNA-Repräsentation (0,5 µg/µl)
      140 µl 10 x DpnII-Puffer
      100 µl DpnII (10 U/µl)
      560 µl Wasser.
   b) Der Restriktionsverdau-Ansatz wurde vor dessen Aufbereitung auf 2 Gefäße aufgeteilt.
      Extraktion: 2 x Phenol/Chloroform (1:1), 1 x Chloroform 100%;
      Fällung: Zugabe von 70 µl 3 M Na-Acetat (pH 5,3), 700 µl 2-Propanol zu jedem Gefäß, 20 min Eis;
      Zentrifugation: 14 min, 14000 rpm, 4°C.
      Waschen des DNA-Pellets mit Ethanol 70% und Resuspension in soviel Wasser, daß eine Konzentration von 0 , 5 µg/µl resultierte.

      Die so erhaltene, DpnII-verdaute (whn +/+)-Haut-cDNA-Repräsentation stellte die in der subtraktiven Hybridisierung einzusetzende Driver-DNA-Population dar.
5. Synthese der Tester-DNA-Population
   a) 20 µg der mit DpnII verdauten (whn -/-)-Haut-cDNA-Repräsentation (= Tester-DNA) wurden in einem TAE-Gel elektrophoretisch aufgetrennt:
      40 µl Tester-DNA (0,5 µg/µl)
      50 µl Te-Puffer
      10 µl 10 x Loading Buffer
      wurden auf ein 1,2% Agarose-TAE-Gel aufgetragen. Es wurde solange Spannung an das Gel gelegt, bis die Bromphenolblau-Komponente des Loading Buffers ungefähr 2 cm weit gewandert war.
   b) Anschließend wurden die Repräsentations-DNA enthaltenden Banden aus dem Gel ausgeschnitten und mit Hilfe des "Agarose Gel DNA Extraction Kits" der Firma Boehringer Mannheim eluiert.
      Die DNA-Extrakte wurden vereinigt, so daß insgesamt 60 µl Lösung erhalten wurden. Die Konzentration dieser Lösung wurde durch Elektrophorese von 5 µl in einem 1% Agarose-Gel abgeschätzt.
   c) Zuletzt erfolgte eine Ligation der Tester-DNA mit dem J-Oligonukleotid-Paar: 2 µg Tester-DNA-Eluat
      6 µl 10 x Ligase Puffer
      4 µl J-Bgl-24 (2 µg/µl)
      4 µl J-Bgl-12 (1 µg/µl)
      x µl Wasser
      57 µl Endvolumen
   d) Überführung des Reaktionsansatzes in Thermocycler:
      1 min: 50°C
      Abkühlen auf 10°C in 1 h (ramp rate:
      0,1°C/9 sec).
   e) Nach Hinzufügen von 3 µl T4 DNA Ligase (1 U/µl) Inkubation bei 16°C über Nacht.
   f) Einstellung der Konzentration der Tester-DNA auf ungefähr 10 ng/µl durch Zugabe von 120 µl Wasser.
6. Subtraktive Hybridisierung
   a) 80 µl Driver-DNA (40 µg) aus Schritt 4. und 40 µl (0,4 µg) verdünnte, mit J-Oligonukleotiden ligierte Tester-DNA aus Schritt 5. wurden in einem Reaktionsgefäß vereinigt und 2 x mit Phenol/Chloroform (1:1) und einmal mit Chloroform 100% extrahiert.
   b) Fällung durch Zugabe von 30 µl 10 M Ammoniumacetat, 380 µl Ethanol 100%; 10 min -70°C.
      Zentrifugation: 14 min, 14000 rpm, 4°C.
      Anschließend: 2 x Waschen des Pellets mit Ethanol 70%, kurze Zentrifugation nach jedem Waschschritt; Trocknen des DNA-Pellets.
   c) Die Resuspension der DNA erfolgte in 4 µl EE x3-Puffer (30 mM EPPS, pH 8,0 bei 20°C (Firma Sigma), 3 mM EDTA) - hierbei wurde ungefähr 2 min auf- und abpipettiert, dann 5 min auf 37°C erwärmt, kurz "gevortext" und zuletzt die Lösung durch Zentrifugieren wieder am Gefäßboden vereinigt. Zuletzt wurde die Lösung mit 35 µl Mineralöl überschichtet.
   d) Überführen des Reaktionsansatzes in Thermocycler:
      5 min: 98°C,
      Abkühlen auf 67°C und sofortige Zugabe von 1 µl 5 M NaCl zur DNA,
      20 h Inkubation bei 67°C.
7. Synthese des ersten Differenzprodukts
   a) Nachdem das Mineralöl möglichst vollständig entfernt worden war, wurde die DNA schrittweise verdünnt:
      1. Zugabe von 8 µl TE (+ 5 µg/µl Hefe-RNA),
      2. Zugabe von 25 µl TE - danach gründliches Mischen,
      3. Zugabe von 362 µl TE - Vortex.
   b) Für jede subtraktive Hybridisierung wurden 4 PCRs angesetzt. Pro Reaktion:
      127 µl Wasser
      20 µl 10 x Puffer
      20 µl 2 mM dNTPs
      5 µl 25 mM Mg-Chlorid
      20 µl verdünnte Hybridisierungslösung (aus Schritt 7a))
   c) PCR-Programm:
      3 min: 72°C
      Zugabe von 1 µl Taq DNA Polymerase (5 U/µl)
      5 min: 72°C
      Zugabe von 2 µl Primer J-Bgl-24 (1 µg/µl) 10 x: 1 min: 95°C
      3 min: 70°C
      zuletzt: 10 min: 72°C; dann Abkühlen auf Raumtemperatur.
   d) Die 4 Reaktionsansätze wurden in einem 1,5 ml-Gefäß vereinigt.
      Extraktion: 2 x Phenol/Chloroform (1:1), 1 x Chloroform 100%.
      Nach Zugabe von 2 µg Glykogen Carrier:
      Fällung mit 75 µl 3 M Na-Acetat (pH 5,3), 800 µl 2-Propanol, 20 min Eis.
      Zentrifugation: 14 min, 14000 rpm, 4°C.
      Waschen des DNA-Pellets mit Ethanol 70%.
      Nach Trocknen der DNA Resuspension in 40 µl Wasser.
   e) 20 µl der resuspendierten DNA aus d) wurden einem "Mung Bean Nuclease-Verdau" (=MBN) unterzogen:
      20 µl DNA
      4 µl 10 x Mung Bean Nuclease Buffer (Fa. NEB)
      14 µl Wasser
      2 µl Mung Bean Nuclease (10 U/µl; Fa. NEB) 35 min, 30°C.

      Die Reaktion wurde durch Zugabe von 160 µl 50 mM Tris-HCl (pH 8,9) und 5-minütige Inkubation bei 98°C abgebrochen. Anschließend wurde das Gefäß bis zum nächsten Schritt auf Eis gesetzt.
   f) Während der MBN-Inkubation wurden 4 weitere PCRs angesetzt (auf Eis):
      127 µl Wasser
      20 µl 2 mM dNTPs
      10 µl 25 mM Mg-Chlorid
      2 µl J-Bgl-24 (1 µg/µl)
      20 µl MBN-verdaute DNA.
   g) PCR-Programm:
      1 min: 95°C
      Abkühlenlassen auf 80°C, Zugabe von 1 µl Taq DNA Polymerase (5 U/µl),
      18 x: 1 min: 95°C 3 min: 70°C,
      zuletzt: 10 min: 72°C; Abkühlenlassen auf 4°C.
   h) Die 4 PCR-Ansätze wurden in einem Gefäß vereinigt.
      Extraktion: 2 x Phenol/Chloroform (1:1), 1 x Chloroform 100%.
      Fällung: 75 µl 3 M Na-Acetat (pH 5,3), 800 µl 2-Propanol, 20 min Eis.
      Zentrifugation: 14 min, 14000 rpm, 4°C.
      Waschen des DNA-Pellets mit Ethanol 70%. Resuspension der DNA in 100 µl Wasser (resultierende Konzentration: 0,5 µg/µl); die auf diese Weise erhaltene Lösung stellte das erste Differenzprodukt dar.
8. Austausch der Oligonukleotidadaptoren des Differenzprodukts
   a) Entfernung der Oligonukleotidadaptoren durch Restriktionsverdau mit DpnII:
      40 µl Differenzprodukt 1 (0,5 µg/µl)
      30 µl 10 x DpnII Puffer
      15 µl DpnII (10 U/µl)
      215 µl Wasser
      2 h 37°C.
   b) Aufarbeitung des Reaktionsansatzes:
      Extraktion: 2 x Phenol/Chloroform (1:1), 1 x Chloroform 100%.
      Fällung: 33 µl 3 M Na-Acetat (pH 5,3), 800 µl Ethanol 100%, 20 min - 20°C.
      Zentrifugation: 14 min, 14000 rpm, 4°C.
      Waschen des Pellets in Ethanol 70% und Resuspension in 40 µl Wasser.
   c) Ligation des Differenzprodukts an N-Bgl-Oligonukleotidadaptorenpaar
      1 µl der aufgearbeiteten DNA-Lösung aus Schritt b) wurde mit 9 µl Wasser zu einer Konzentration von 50 ng/µl verdünnt; 4 µl dieser Lösung wurden in folgender Reaktion eingesetzt:
      4 µl DpnII verdautes Differenzprodukt 1 (200 ng)
      6 µl 10 x Ligase Puffer
      2,5 µl N-Bgl-24 (3,5 µg/µl)
      2 µl N-Bgl-12 (2 µg/µl)
      42,5 µl Wasser.
   d) Nach Überführen des Reaktionsansatzes in Thermocycler:
      1 min: 50°C,
      Abkühlenlassen innerhalb einer Stunde auf 10°C (ramp rate: 0,1°C/9 sec).
   e) Nach Hinzugeben von 3 µl T4 DNA Ligase (1 µ/µl), Inkubation bei 16°C über Nacht.
9. Synthese des 2. Differenzprodukts
   Der Ligationsansatz aus Schritt 8e) wurde durch Zugabe von 100 µl Wasser auf eine Konzentration von 1,25 ng/µl verdünnt. 40 µl dieser Verdünnung (50 ng) wurden mit 80 µl Driver-DNA (siehe Punkt 4.) gemischt und erneut gemäß den Schritten 6. bis 8. behandelt. Beim Wechsel der Oligonukleotidadaptoren (Schritt 8.) wurden dieses Mal die J-Bgl-Oligonukleotide an das neu entstandene Differenzprodukt 2 ligiert.
10. Synthese des 3. Differenzprodukts
   Die Konzentration des mit den J-Bgl-Oligos ligierten Differenzprodukts 2 wurde auf eine Konzentration von 1 ng/µl reduziert. 10 µl dieser Lösung wurden wiederum mit 990 µl Wasser (+ 30 µg Hefe-RNA) verdünnt, so daß die Konzentration nunmehr 10 pg/µl betrug. Die subtraktive Hybridisierung wurde mit 100 pg (10 µl) J-ligiertem Differenzprodukt 2 und 40 µg (80 µl) Driver-DNA aus Schritt 4.) durchgeführt.
   Ansonsten wurde wie beim 1. und 2. Differenzprodukt nach den Schritten 6. bis 8. vorgegangen. Eine Ausnahme bildete die PCR nach der MBN-Reaktion (Punkt 7.g) - hier wurden nur 18 statt 22 Cyclen durchgeführt.
11. Klonierung des 3. Differenzprodukts
   Das 3. Differenzprodukt wurde zunächst einem Restriktionsverdau mit DpnII unterzogen, wodurch die Oligonukleotidadaptoren entfernt wurden. Das Reaktionsprodukt wurde anschließend auf ein TAE-Gel aufgetragen und elektrophoretisch getrennt. Die getrennten DNA-Banden wurden aus dem Gel ausgeschnitten, die DNA eluiert und in einen mit BamHI geschnittenen Vektor (pBS Not) kloniert.
12. Charakterisierung der Differenzprodukte
   Um zu bestätigen, daß es sich bei den klonierten DNA-Fragmenten nicht um Methodenartefakte handelte, sondern um Sequenzen, die tatsächlich in den untersuchten DNA-Repräsentationen enthalten waren, wurden Southern Analysen durchgeführt, bei denen die untersuchten cDNA-Repräsentationen mit den radioaktiv markierten Klonierungsprodukten hybridisiert wurden.
   Anschließend wurden diejenigen DNA-Fragmente, die sich in der Southern Analyse als "echte" Differenzprodukte erwiesen hatten, mittels Northern Hybridisierungen untersucht: es wurden RNAs aus den untersuchten Geweben (whn +/+)-HautcDNA und (whn -/-)-Haut-cDNA) geblottet und mit den radioaktiv markierten Klonierungsprodukten hybridisiert. Hierdurch wurde die differentielle Expression dieser Sequenzen in den untersuchten Geweben bestätigt. Eine Analyse der Sequenzen ergab, daß in nu/nu-Mäusen (Alopezie aufweisende Mäuse) das Ha3-Gen nicht exprimiert wird (vgl. Fig. 1).

### Beispiel 2: Expression von Haarkeratin- und whn-Genen in normalen und Alopezie aufweisenden Mäusen.

Aus der Haut von unterschiedlich alten normalen (whn +/+) und nackten (whn -/-) Mäusen wurde RNA isoliert, in Agarose-Gelen elektrophoretisch aufgetrennt, auf Filter transferiert und mit genspezifischen Sonden hybridisiert.

Die verwendeten Sonden waren wie folgt:
mHa1: Nukleotide 1331 - 1551; Genbank "Accession"-Nr. M27734
mHa3: Nukleotide 1007 - 1204; Genbank "Accession"-Nr. X75650
mHa4: Nukleotide 1303 - 1542, vgl. Bertolino, A.P. et al., J. Invest. Dermatol. 94, (1990) 297 - 303 whn: Nukleotide 1141 - 1374; Genbank "Accession"-Nr. X81593

Es zeigte sich, daß Haarkeratin- und whn-Gene in Alopezie aufweisenden Mäusen nicht bzw. nur schwach exprimiert werden.

### Beispiel 3: Nachweis der Expressions-Induktion des Ha3-Gens durch das Genprodukt des whn-Gens.

Ein am N-terminalen Epitop "getaggtes" whn-Gen wurde in den Expressionsvektor pTRE (Clontech) inseriert. Das erhaltene DNA-Konstrukt wurde für eine transiente Transfektion der Hela Tet-On Zell-Linie (Clontech) mittels des Calciumphosphat-Copräzipitations-Verfahrens verwendet. Die Zellen wurden unmittelbar danach mit 5 µg/ml Docyclin behandelt. 24 h später wurde 1 mM Natriumbutyrat zugegeben. 48 h nach Transfektion wurden die Zellen geerntet und einem RT-PCR-Verfahren unterzogen. Die im PCR-Verfahren verwendeten Primer waren wie folgt:
hHa3: 5'-CTGATCACCAACGTGGAGTC-3', 5'-TACCCAAAGGTGTTGCAAGG-3'.

Das PCR-Verfahren umfaßte 35-40 Zyklen mit jeweils 30 sec bei 95°C, 30 sec bei 58°C und 1 min bei 72°C.

Es zeigte sich, daß durch die Expression des whn-Gens eine Expression des Ha3-Gens induziert wurde. Parallele Kontrollen, in denen keine Transfektion mit dem whn-Gen erfolgte, führten zu keiner Induktion der Ha3-Gen-Expression.

### Beispiel 4: Herstellung eines erfindungsgemäßen Systems

Aus einer BAC-Bibliothek der Firma Genome Systems (St. Louis, Missouri, USA;) wurde ein mit BAC-whn bezeichneter BAC-Klon isoliert, der das gesamte whn-Gen der Maus umfaßt (vgl. Schorpp, M. et al., Immunogenetics 46, (1997), 509-515).

Ferner wurde ein mit pMB096-whn-GFP bezeichneter Shuttle-Vektor verwendet, der das whn-Gen der Maus enthielt, wobei bei diesem in Exon3 das Reporter-Gen GFP vorlag (vgl. Nehls, M. et al., Science 272, (1996), 886-889).

BAC-whn wurde zur Transformation des recA⁺ E.coli-Stammes CBTS verwendet. Die Transformation erfolgte durch Elektroporation. Klone wurden isoliert und mit pMB096-whn-GFP mittels Elektroporation transformiert. Es erfolgte eine homolge Rekombination zwischen dem BAC-Klon und dem Shuttle-Vektor im Bereich des whn-Gens, wodurch ein mit BAC-whn-GFP bezeichneter Vektor erhalten wurde. Dieser wies im whn-Gen das Reporter-Gen GFP auf.

BAC-whn-GFP wurde zur Transfektion von COS-Zellen verwendet. Die Tranfektion erfolgte mittels des Calciumphosphat-Copräzipitations-Verfahrens. Es wurden COS-Zellen erhalten, die für ein Fusionsgen aus whn und GFP kodierten.

Es zeigte sich, daß diese Zellen geeignet waren, Substanzen zu identifizieren, welche die Genexpression von whn induzieren konnten. Solche Substanzen eigneten sich zur Hemmung von Alopezie.

## Patentansprüche

1. Verwendung von Haarkeratinen zur Herstellung eines Arzneimittels zur Erhöhung der zellulären Menge von Haarkeratinen zur Hemmung von Alopezie.

2. Verwendung nach Anspruch 1, wobei die Haarkeratine in Form von sie exprimierender DNA vorliegen.

3. Verwendung nach Anspruch 1 oder 2, wobei die Haarkeratine Ha1, Ha2, Ha3 und Ha4 umfassen.

4. Verwendung nach einem der Ansprüche 1 - 3, wobei den Zellen ferner das Genprodukt des whn-Gens und/oder eine es exprimierende DNA zugegeben wird.

5. Verfahren zur Identifizierung von Alopezie hemmenden Stoffen, bei dem die Erhöhung der zellulären Menge von Haarkeratinen und/oder die Erhöhung ihrer Genexpression und/oder die Erhöhung des Genprodukts des whn-Gens bestimmt wird, wobei Zellen verwendet werden, in denen ein oder mehrere exprimierende Haarkeratin-Gene fusioniert mit einem Reporter-Gen vorliegen.

6. Verfahren nach Anspruch 5, wobei die Haarkeratine, Ha1, Ha2, Ha3 und Ha4 umfassen.

7. Verfahren nach Anspruch 5 oder 6, wobei in den Zellen ferner eine das whn-Gen exprimierende DNA fusioniert mit einem Reporter-Gen vorliegt.

8. Verfahren nach einem der Ansprüche 5 - 7, wobei das Reporter-Gen für ein Enzym kodiert.

9. Verfahren nach einem der Ansprüche 5 - 7, wobei das Reporter-Gen für ein fluoreszierendes Protein kodiert.

10. Verfahren nach einem der Ansprüche 5 - 9, wobei die Fusionsgene extrachromosomal vorliegen.

11. Verfahren nach einem der Ansprüche 5 - 9, wobei die Fusionsgene im Zell-Genom integriert sind.

## Claims

1. Use of hair keratins for the production of a medicament to increase the cellular amount of hair keratins for the inhibition of alopecia.

2. Use according to claim 1, wherein the hair keratins are available in the form of DNA expressing the same.

3. Use according to claim 1 or 2, wherein the hair keratins comprise Ha1, Ha2, Ha3 and Ha4.

4. Use according to any of claims 1 to 3, wherein the cells are also provided with the gene product of the whn gene and/or a DNA expressing the same.

5. A process for identifying alopecia inhibiting substances, comprising determining an increase in the cellular amount of hair keratins and/or an increase in their gene expression and/or an increase in the gene product of the whn gene, cells being used comprising one or several expressing hair keratin genes which are fused to a reporter gene.

6. The process according to claim 5, wherein the hair keratins comprise Ha1, Ha2, Ha3 und Ha4.

7. The process according to claim 5 or 6, wherein a DNA expressing the whn gene is also present in the cells in a form fused to a reporter gene.

8. The process according to any of claims 5 to 7, wherein the reporter gene codes for an enzyme.

9. The process according to any of claims 5 to 7, wherein the reporter gene codes for a fluorescent protein.

10. The process according to any of claims 5 to 9, wherein the fusion genes are available in an extrachromosomal form.

11. The process according to any of claims 5 to 9, wherein the fusion genes are integrated in the cell genome.

## Revendications

1. Utilisation de kératine des cheveux pour la production d'un médicament destiné à élever la quantité cellulaire de kératine des cheveux en vue d'inhiber l'alopécie.

2. Utilisation suivant la revendication 1, dans laquelle la kératine des cheveux est présente sous forme de l'ADN qui l'exprime.

3. Utilisation suivant la revendication 1 ou 2, dans laquelle la kératine des cheveux comprend Ha1, Ha2, Ha3 et Ha4.

4. Utilisation suivant les revendications 1 à 3, dans laquelle le produit génique du gène whn et/ou un ADN qui l'exprime sont en outre ajoutés aux cellules.

5. Méthode d'identification de substances inhibant l'alopécie, dans laquelle sont déterminées l'élévation de la quantité cellulaire de kératine des cheveux et/ou l'élévation de son expression génique et/ou l'élévation du produit génique du gène whn, les cellules utilisées étant des cellules dans lesquelles un ou plusieurs gènes d'expression de la kératine fusionnés avec un gène rapporteur sont présents.

6. Méthode suivant la revendication 5, dans laquelle la kératine des cheveux comprend Ha1, Ha2, Ha3 et Ha4.

7. Méthode suivant la revendication 5 ou 6, dans laquelle un ADN exprimant le gène whn fusionné avec un gène rapporteur est en outre présent dans les cellules.

8. Méthode suivant l'une des revendication 5 à 7, dans laquelle le gène rapporteur code pour un enzyme.

9. Méthode suivant l'une des revendications 5 à 7, dans laquelle le gène rapporteur code pour une protéine fluorescente.

10. Méthode suivant l'une des revendications 5 à 9, dans laquelle la présence des gènes de fusion est extrachromosomique.

11. Méthode suivant l'une des revendications 5 à 9, dans laquelle les gènes de fusion sont intégrés au génome des cellules.
